# EUROPÄISCHE PATENTSCHRIFT

(11) **EP 1 979 508 B1**
(45) Veröffentlichungstag und Bekanntmachung des Hinweises auf die Patenterteilung: **28.10.2009**
(21) Anmeldenummer: 07702644.1
(22) Anmeldetag: 10.01.2007
(51) Int. Cl.: C23F 11/16, C07C 323/59, C09K 8/54

(54) **KORROSIONSINHIBITOREN MIT ERHÖHTER BIOLOGISCHER ABBAUBARKEIT UND VERMINDERTER TOXIZITÄT**
CORROSION INHIBITORS HAVING INCREASED BIODEGRADABILITY AND REDUCED TOXICITY
INHIBITEURS DE CORROSION AYANT UNE BIODEGRADABILITE AMELIOREE ET UNE TOXICITE MOINDRE

(30) Priorität: 20.01.2006 DE 102006002784
(43) Veröffentlichungstag der Anmeldung: 15.10.2008
(73) Patentinhaber: Clariant Finance (BVI) Limited, Road Town, Tortola (VG)
(72) Erfinder: LEINWEBER, Dirk, 65779 Kelkheim (DE); FEUSTEL, Michael, 55278 Köngernheim (DE)
(74) Vertreter: Mikulecky, Klaus
(86) Internationale Anmeldenummer: PCT/EP2007/000140
(87) Internationale Veröffentlichungsnummer: WO 2007/087960

(56) Entgegenhaltungen:
- JP-A- 8 337 562
- JP-A- 8 337 563
- US-A- 6 117 364
- DATABASE WPI Week 200048 Derwent Publications Ltd., London, GB; AN 2000-527412 XP002428137 -& JP 2000 169870 A (ASAHI DENKA KOGYO KK) 20. Juni 2000 (2000-06-20)
- DATABASE WPI Week 197439 Derwent Publications Ltd., London, GB; AN 1974-68604V XP002428138 -& JP 49 026145 A (AJINOMOTO KK) 8. März 1974 (1974-03-08) in der Anmeldung erwähnt

## Beschreibung

Die vorliegende Erfindung betrifft ein Verfahren zur Korrosionsinhibierung an und in Einrichtungen zur Förderung und Transport von Kohlenwasserstoffen in der Erdölförderung und -verarbeitung, indem man dem korrosiven System ein Salz aus einer Stickstoffbase und einem N-Acylmethionin zusetzt.

In technischen Prozessen, bei denen Metalle mit Wasser oder auch mit Öl-Wasser-Zweiphasensystemen in Kontakt kommen, besteht die Gefahr der Korrosion. Diese ist besonders ausgeprägt, wenn die wässrige Phase wie bei Erdölgewinnungs- und Verarbeitungsprozessen stark salzhaltig oder durch gelöste Sauergase, wie Kohlendioxid bzw. Schwefelwasserstoff, azid ist. Daher sind die Ausbeutung einer Lagerstätte und die Verarbeitung von Erdöl ohne spezielle Additive zum Schutz der eingesetzten Ausrüstungen nicht möglich.

Geeignete Korrosionsschutzmittel für die Erdölförderung und -verarbeitung sind zwar schon seit langem bekannt, jedoch aus Gründen des Umweltschutzes für Offshore-Anwendungen zukünftig inakzeptabel.

Als typische Korrosionsinhibitoren des Standes der Technik besitzen Amide, Amidoamine bzw. Imidazoline von Fettsäuren und Polyaminen eine äußerst gute Öllöslichkeit und sind somit in der korrosiven Wasserphase aufgrund schlechter Verteilungsgleichgewichte (Partitioning) nur in geringer Konzentration vorhanden. Demgemäß müssen diese Produkte trotz ihrer schlechten biologischen Abbaubarkeit in hoher Dosierung eingesetzt werden.

Quartäre Alkylammoniumverbindungen (Quats) stellen alternative Korrosionsschutzmittel des Standes der Technik dar, die neben den korrosionsinhibierenden auch biostatische Eigenschaften besitzen können. Trotz einer verbesserten Wasserlöslichkeit zeigen die Quats, zum Beispiel im Vergleich zu den Imidazolinen, eine deutlich reduzierte Filmpersistenz und führen daher ebenfalls nur in höherer Dosierung zu einem effektiven Korrosionsschutz. Die starke Algentoxizität und die mäßige biologische Abbaubarkeit beschränken den Einsatz von Quats immer mehr auf ökologisch unsensible Anwendungsgebiete.

US-4 240 823 beschreibt N-Acyl-Methionin Derivate, die als Wachstumsregulatoren im Bereich des Pflanzenschutzes eingesetzt werden. Aminsalze von N-Acyl-Methionin Derivaten werden nicht beschrieben.

In JP-A-8 337 562 und JP-A-8 337 563 werden N-Acyl-Aminosäuren und ihre Alkalimetallsalze beschrieben, die auch als Korrosionsinhibitoren eingesetzt werden können. Es werden keine Aminsalze von N-Acyl-Methionin Derivaten beschrieben.

In JP-A-49 026 145 werden N-Acyl-Aminosäure Alkalimetallsalze beschrieben, die als Korrosionsinhibitoren eingesetzt werden können. Als Beispiel wird N-Lauroylglycin Natriumsalz genannt. Aminsalze von N-Acyl Methionin Derivaten werden nicht beschrieben.

Nachteilig an den Verbindungen des Standes der Technik ist jedoch, dass ihre Wirksamkeit nicht ausreichend ist und dass sie stark zum Schäumen neigen.

Aufgabe der vorliegenden Erfindung war es, neue Korrosionsinhibitoren zu finden, die bei konstant gutem oder verbessertem Korrosionsschutz neben einer guten Wasserlöslichkeit, geringer Schaumbildung auch eine verbesserte biologische Abbaubarkeit und niedrigere Toxizität im Vergleich zu den Korrosionsinhibitoren des Standes der Technik bieten.

Es wurde nun überraschenderweise gefunden, dass N-Acyl-Methionin-Ammoniumsalze eine ausgezeichnete Wirkung als Korrosionsinhibitoren und eine geringe Schaumbildungstendenz aufweisen, sowie eine gute biologische Abbaubarkeit und verminderte Toxizität zeigen.

Gegenstand der Erfindung ist somit die Verwendung von Salzen aus Verbindungen der Formel (1) 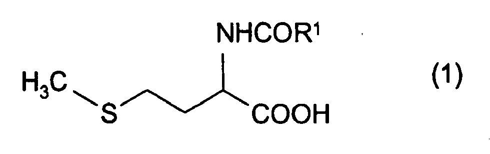 und Aminen der Formel (2) 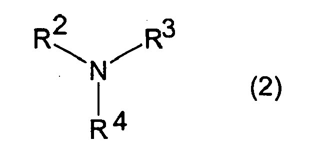 worin
- R¹: C₁- bis C₃₀-Alkyl, C₂- bis C₃₀-Alkenyl, C₆- bis C₃₀-Aryl- oder C₇- bis C₃₀-Alkylaryl,
- R²: C₁- bis C₃₀-Alkyl, C₂- bis C₃₀-Alkenyl, C₆- bis C₃₀-Aryl- oder C₇- bis C₃₀-Alkylaryl, oder einen gegebenenfalls Heteroatome enthaltenden organischen Rest mit 1 bis 30 Kohlenstoffatomen, und,
- R³, R⁴: unabhängig voneinander Wasserstoff, C₁- bis C₃₀-Alkyl, C₂- bis C₃₀- Alkenyl, C₆- bis C₃₀-Aryl- oder C₇- bis C₃₀-Alkylaryl, oder einen gegebenenfalls Heteroatome enthaltenden organischen Rest mit 1 bis 30 Kohlenstoffatomen bedeuten, wobei R³ und R⁴ auch unter Einschluss des Stickstoffatoms einen Cyclus mit 5 bis 7 Ringatomen bilden können,
als Korrosionsinhibitoren.

Korrosive Systeme im Sinne dieser Erfindung sind bevorzugt flüssig/flüssig- bzw. flüssig/gasförmig-Mehrphasensysteme, bestehend aus Wasser und Kohlenwasserstoffen, die in freier und/oder gelöster Form korrosive Bestandteile, wie Salze und Säuren, enthalten. Die korrosiven Bestandteile können auch gasförmig sein, wie etwa Schwefelwasserstoff und Kohlendioxid.

Kohlenwasserstoffe im Sinne dieser Erfindung sind organische Verbindungen, die Bestandteile des Erdöls/Erdgases sind, und deren Folgeprodukte. Kohlenwasserstoffe im Sinne dieser Erfindung sind auch leichtflüchtige Kohlenwasserstoffe, wie beispielsweise Methan, Ethan, Propan, Butan. Für die Zwecke dieser Erfindung zählen dazu auch die weiteren gasförmigen Bestandteile des Erdöls/Erdgases, wie etwa Schwefelwasserstoff und Kohlendioxid.

Ein weiterer Gegenstand der Erfindung ist die Verwendung der Verbindungen aus Formeln (1) und (2) in Metallbearbeitungsmittel. Hierbei bieten die erfindungsgemäßen Verbindungen auch bei starker mechanischer Belastung, wie beim Schleifen, Schneiden und Bohren von Metallwerkstücken einen sehr guten Korrosionsschutz.

In einer bevorzugten Ausführungsform der Erfindung ist die Verbindung der Formel (2) ein cyclisches Amin der Formel (3) 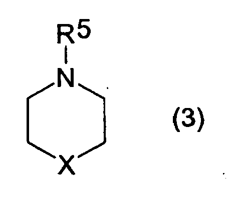 worin
- R⁵: Wasserstoff oder einen gegebenenfalls Heteroatome enthaltenden C₁₋₃₀ Alkylrest, und
- X: C, O oder N bedeuten,

R¹ steht vorzugsweise für eine Alkyl- oder Alkenylgruppe mit 2 bis 24 Kohlenstoffatomen, insbesondere für eine Alkyl- oder Alkenylgruppe mit 8 bis 18 Kohlenstoffatomen.

R² steht für einen organischen Rest, der 1 bis 30 C-Atome und gegebenenfalls Heteroatome enthalten kann. Enthält R² Heteroatome, so handelt es sich vorzugsweise um Stickstoff- und/oder Sauerstoffatome. In einer bevorzugten Ausführungsform steht R² für -CH₂-CH₂-OH.

R³ und R⁴ können unabhängig voneinander beliebige organische Reste sein, die Wasserstoff oder 1 bis 30 C-Atome und gegebenenfalls Heteroatome enthalten. Enthalten R³ und/oder R⁴ Heteroatome, so handelt es sich vorzugsweise um Stickstoff- und/oder Sauerstoffatome. In einer bevorzugten Ausführungsform stehen einer oder beide Reste R³ und R⁴ für -CH₂-CH₂-OH. Die Formel (2) stellt damit vorzugsweise Mono-, Di- oder Triethanolamin dar. Erfindungsgemäß ist auch die Verwendung alkoxylierte Alkanolamine, beispielsweise von ethoxyliertem N,N-Dibutylaminoethanol.

In einer weiteren bevorzugten Ausführungsform der Erfindung steht R¹ für C₂- bis C₃₀-Alkyl, C₂- bis C₃₀-Alkenyl, C₆- bis C₃₀-Aryl- oder C₇- bis C₃₀-Alkylaryl, und R² für C₁- bis C₃₀-Alkyl, C₂- bis C₃₀-Alkenyl, C₆- bis C₃₀-Aryl- oder C₇- bis C₃₀-Alkylaryl, oder einen gegebenenfalls Stickstoffatome enthaltenden organischen Rest mit 1 bis 30 Kohlenstoffatomen

Die erfindungsgemäßen Verbindungen können alleine oder in Kombination mit anderen bekannten Korrosionsinhibitoren eingesetzt werden. Im Allgemeinen wird man so viel des erfindungsgemäßen Korrosionsinhibitors einsetzen, dass man unter den gegebenen Bedingungen einen ausreichenden Korrosionsschutz erhält.

Bevorzugte Einsatzkonzentrationen der Korrosionsinhibitoren bezogen auf die reinen erfindungsgemäßen Salze sind 5 bis 5000 ppm, bevorzugt 10 bis 1000, insbesondere 15 bis 150 ppm.

Besonders geeignet als Korrosionsinhibitoren sind auch Mischungen der erfindungsgemäßen Salze mit anderen Korrosionsinhibitoren und/oder des Standes der Technik.

Besonders geeignet als Korrosionsinhibitoren und somit eine bevorzugte Ausführungsform dieser Erfindung sind Mischungen der erfindungsgemäßen Salze mit Amidoaminen und/oder Imidazolinen aus Fettsäuren und Polyaminen und deren Salzen, quartären Ammoniumsalzen, oxethylierten/oxpropylierten Aminen, Amphoglycinaten und -propionaten, Betainen oder Verbindungen beschrieben in DE-A-199 30 683.

Die Herstellung von N-Acyl-Methionin-Derivaten erfolgt durch Acylierung von Methionin mittels eines Carbonsäurechlorids oder -anhydrids in Anwesenheit einer Base (z.B. Natriumhydroxid). Durch anschließende Neutralisation, Abtrennung der wässrigen Salzlösung und Umsetzung mit Aminen sind die erfindungsgemäßen N-Acyl Methionin Ammoniumsalze herstellbar.

Bevorzugt wird hierzu aus ökonomischen Gründen DL-Methionin eingesetzt, die enantiomerenreinen Formen können aber ebenfalls verwendet werden.

Zur Acylierung werden bevorzugt C₈₋₁₈ Alkyl- bzw. Alkenylchloride eingesetzt, wie beispielsweise Octansäurechlorid, Decansäurechlorid, Dodecansäurechlorid, Kokosfettsäurechlorid oder Ölsäurechlorid.

Bevorzugt verwendete Amine der Formel (2) sind beispielsweise Methylamin, Ethylamin, Propylamin, Butylamin, Cyclohexylamin, Dicyclohexylamin, Laurylamin, Cocosfettamin, Stearylamin, Dimethylamin, Diethylamin, Dipropylamin, Dibutylamin, 3-Dimethylaminopropylamin, 3-Diethylaminopropylamin, 3-Morpholinopropylamin, Diethylentriamin, Triethylentetramin, Tetraethylenpentamin, Monoethanolamin, Diethanolamin, Triethanolamin, Morpholin, Morpholin Produktionsrückstände, N,N-Dimethylaminoethanol, N,N-Diethylaminoethanol, N,N-Dibutylaminoethanol, 3-Dimethylaminopropanol, N-Hydroxyethylmorpholin, 3-Aminopropanol, Isopropanolamin, 2-(2-Aminoethoxy)-ethanol und Cyclohexylamino-N,N-diethanol, Aminoethylmorpholin und Aminoethylpiperazin.

### Beispiele:

### Allgemeine Vorschrift für die Herstellung von N-Acyl-Methionin-Ammoniumsalzen

In einer Standard-Rührapparatur werden 1 mol DL-Methionin in 300 mL Wasser mit 50 %iger Natronlauge neutralisiert. Zu der gebildeten Lösung werden bei 15-20°C 1 mol Carbonsäurechlorid zudosiert, wobei der pH-Wert durch parallele Dosierung von 15 %iger Natronlauge bei 10-13 gehalten wird. Die Reaktionslösung wird 3 h bei Raumtemperatur nachgerührt. Dann wird das gebildete N-Acyl DL-Methionin-Natriumsalz mit 32 %iger Salzsäure neutralisiert, von der wässrigen Salzphase abgetrennt und getrocknet. Anschließend wird das N-Acyl-DL-Methionin durch Zugabe einer äquimolaren Menge des entsprechenden Amins in das N-Acyl-DL-Methionin-Ammoniumsalz überführt. Das erhaltene Produkt wird mittels Säurezahl (SZ) und Basen-Stickstoff (bas.-N) charakterisiert. Prozentangaben sind Gewichtsprozente bezogen auf das Gewicht des erfindungsgemäßen Salzes.

### Beispiel 1 N-Octyl-DL-Methionin-Monoethanolammoniumsalz

Aus 162,7 g Octansäurechlorid, 117,2 g DL-Methionin und 61,1g Monoethanolamin wurden 304,5,1 g N-Octyl-DL-Methionin-Monoethanolammoniumsalz
mit SZ = 184 mg KOH/g und bas.-N = 4,58 % erhalten.

### Beispiel 2 N-Octyl-DL-Methionin-Triethanolammoniumsalz

Aus 162,7 g Octansäurechlorid, 117,2 g DL-Methionin und 149,2 g Triethanolamin wurden 392,0 g N-Octyl-DL-Methionin-Triethanolammoniumsalz mit SZ = 143 mg KOH/g und bas.-N = 3,55 % erhalten.

### Beispiel 3 N-Dodecyl-DL-Methionin-Cyclohexylammoniumsalz

Aus 218,8 g Dodecansäurechlorid, 117,2 g DL-Methionin und 99,2 g Cyclohexylamin wurden 398,4 g N-Dodecyl-DL-Methionin-Cyclohexylammoniumsalz mit SZ = 140 mg KOH/g und bas.-N = 3,49 % erhalten.

### Beispiel 4 N-Dodecyl-DL-Methionin-Dibutylammoniumsalz

Aus 218,8 g Dodecansäurechlorid, 117,2 g DL-Methionin und 129,3 g Dibutylamin wurden 428,3 g N-Dodecyl-DL-Methionin-Dibutylammoniumsalz mit SZ = 130 mg KOH/g und bas.-N = 3,23 % erhalten.

### Beispiel 5 N-Kokoyl-DL-Methionin-Morpholiniumsalz

Aus 225,3 g Kokosfettsäurechlorid, 117,2 g DL-Methionin und 87,1 g Morpholin wurden 392,0 g N-Kokoyl-DL-Methionin-Morpholiniumsalz mit SZ = 142 mg KOH/g und bas.-N = 3,55 % erhalten.

### Beispiel 6 N-Kokoyl-DL-Methionin-N,N-Diethyl-(2-hydroxyethyl)ammoniumsalz

Aus 225,3 g Kokosfettsäurechlorid, 117,2 g DL-Methionin und 117,2 g N,N-Diethylaminoethanol wurden 419,5 g N-Kokoyl-DL-Methionin-N,N-Diethyl-(2-hydroxyethyl)ammoniumsalz mit SZ = 134 mg KOH/g und bas.-N = 3,30 % erhalten.

### Beispiel 7 N-Oleyl-DL-Methionin-2-(2-Hydroxyethoxy)ethylammoniumsalz

Aus 300,9 g Ölsäurechlorid, 117,2 g DL-Methionin und 105,4 g 2-(2-Aminoethoxy)-ethanol wurden 482,7 g N-OleylDL-Methionin-2-(2-Hydroxyethoxy)-ethylammoniumsalz mit SZ = 116 mg KOH/g und bas.-N = 2,87 % erhalten.

### Beispiel 8 N-Oleyl-DL-Methionin-Triethanolammoniumsalz

Aus 300,9 g Ölsäurechlorid, 117,2 g DL-Methionin und 149,2 g Triethanolamin wurden 526,0 g N-Oleyl-DL-Methionin-Triethanolammoniumsalz mit SZ = 106 mg KOH/g und bas.-N = 2,64 % erhalten.

### Wirksamkeit der erfindungsgemäßen Verbindungen als Korrosionsinhibitoren

Die erfindungsgemäßen Verbindungen wurden als Korrosionsinhibitoren im Shell-Wheel-Test geprüft. Coupons aus C-Stahl (DIN 1.1203 mit 15 cm² Oberfläche) wurden in eine Salzwasser/Petroleum-Mischung (9:1,5 %ige NaCl-Lösung mit Essigsäure auf pH 3,5 gestellt) eingetaucht und bei einer Umlaufgeschwindigkeit von 40 rpm bei 70°C 24 Stunden diesem Medium ausgesetzt. Die Dosierung des Inhibitors betrug 50 ppm einer 40 % Lösung des Inhibitors. Die Schutzwerte wurden aus der Massenabnahme der Coupons, bezogen auf einen Blindwert, berechnet.

In den folgenden Tabellen bezeichnet "Vergleich 1" ein handelsübliches Rückstandsamin - Quat auf Basis Dikokosalkyl-dimethylammoniumchlorid und "Vergleich 2" ein Beispiel aus JP 49026145 (N-Lauroylglycin Natriumsalz, Korrosionsinhibitor des Standes der Technik) und "Vergleich 3" ein Beispiel aus JP-8 337 562 (N-Myristoyl L-asparaginsäure Dinatriumsalz, Korrosionsinhibitor des Standes der Technik).

**Tabelle 1: (Shell-Wheel-Test)**

| Beispiel | Korrosionsinhibitor | ø Schutz % |
|---|---|---|
| Vergleich 1 | Standard-Quat | 36 |
| Vergleich 2 | N-Lauroylglycin Natriumsalz | 45 |
| Vergleich 3 | N-Myristoyl L-asparaginsäure Dinatriumsalz | 38 |
| 9 | Verbindung aus Beispiel 1 | 63 |
| 10 | Verbindung aus Beispiel 2 | 72 |
| 11 | Verbindung aus Beispiel 3 | 78 |
| 12 | Verbindung aus Beispiel 4 | 80 |
| 13 | Verbindung aus Beispiel 5 | 85 |
| 14 | Verbindung aus Beispiel 6 | 83 |
| 15 | Verbindung Aus Beispiel 7 | 82 |
| 16 | Verbindung aus Beispiel 8 | 85 |

Die Produkte wurden außerdem im LPR-Test (Testbedingungen analog ASTM D 2776) geprüft.

**Tabelle 2: (LPR-Test)**

| Beispiel | Korrosionsinhibitor | Schutz nach [%] | | |
|---|---|---|---|---|
| | | 10 min | 30 min | 60 min |
| Vergleich 1 | Standard-Quat | 53,9 | 61,2 | 73,7 |
| Vergleich 2 | N-Lauroylglycin Natriumsalz | 15,4 | 35,2 | 42,9 |
| Vergleich 3 | N-Myristoyl L-asparaginsäure Dinatriumsalz | 20,0 | 42,6 | 47,1 |
| 17 | Verbindung aus Beispiel 1 | 60,5 | 75,3 | 88,4 |
| 18 | Verbindung aus Beispiel 2 | 62,9 | 76,1 | 90,0 |
| 19 | Verbindung aus Beispiel 3 | 76,4 | 88,4 | 97,2 |
| 20 | Verbindung aus Beispiel 4 | 74,8 | 87,3 | 96,8 |
| 21 | Verbindung aus Beispiel 5 | 90,3 | 94,2 | 98,9 |
| 22 | Verbindung aus Beispiel 6 | 92,0 | 96,7 | 99,0 |
| 23 | Verbindung aus Beispiel 7 | 78,5 | 92,9 | 98,5 |
| 24 | Verbindung aus Beispiel 8 | 80,1 | 94,5 | 98,6 |

Wie aus den obigen Testresultaten zu erkennen ist, weisen die erfindungsgemäßen Produkte sehr gute Korrosionsschutzeigenschaften bei niedriger Dosierung auf und übertreffen die Wirksamkeit der Inhibitoren des Standes der Technik deutlich.

**Tabelle 3 (Schüttel-Schaumtest):**

| | | |
|---|---|---|
| Die Schaumeigenschaften wurden mit der Schüttelschaum-Methode überprüft. Dazu wurden 50 ml einer 3 %igen wässrigen Lösung des entsprechenden Korrosionsinhibitors in VE-Wasser in einem verschlossenen 100 ml - Messzylinder innerhalb von 10 Sekunden 20-mal geschüttelt. Für die Beurteilung des Schaumverhaltens wurden nach Beendigung des Schüttelns das Gesamtvolumen der Lösung (Schaumhöhe) und die Schaumzerfallzeit (Zeit bis Erreichen des Ausgangsvolumens von 50 ml) herangezogen. Im Allgemeinen ist dieses Prüfverfahren mäßig reproduzierbar, eignet sich jedoch hervorragend für eine tendenzielle Abschätzung des Schaumverhaltens in schwach schäumend, schäumend oder stark schäumend. | | |

| Beispiel | Korrosionsinhibitor | Schaumverhalten |
|---|---|---|
| Vergleich 1 | Standard-Quat | stark schäumend |
| Vergleich 2 | N-Lauroylglycin Natriumsalz | stark schäumend |
| Vergleich 3 | N-Myristoyl L-asparaginsäure Dinatriumsalz | stark schäumend |
| 17 | Verbindung aus Beispiel 1 | schwach schäumend |
| 18 | Verbindung aus Beispiel 2 | schwach schäumend |
| 19 | Verbindung aus Beispiel 3 | schwach schäumend |
| 20 | Verbindung aus Beispiel 4 | schwach schäumend |
| 21 | Verbindung aus Beispiel 5 | schäumend |
| 22 | Verbindung aus Beispiel 6 | schäumend |
| 23 | Verbindung aus Beispiel 7 | schwach schäumend |
| 24 | Verbindung aus Beispiel 8 | schwach schäumend |

Tabelle 3 zeigt, dass die erfindungsgemäßen Verbindungen eine deutlich geringere Schaumbildungstendenz aufweisen als die Verbindungen aus dem Stand der Technik.

**Tabelle 4: Biologische Abbaubarkeit (OECD 306) und Toxizität (EC₅₀ Skeletonema Costatum)**

| Beispiel | Korrosionsinhibitor | Biologische Abbaubarkeit [%] | Toxizität EC₅₀ [mg/L] |
|---|---|---|---|
| Vergleich 1 | Standard-Quat | 15,2 | 0,57 |
| Vergleich 2 | N-Lauroylglycin Natriumsalz | 44,5 | 8,5 |
| Vergleich 3 | N-Myristoyl L-asparaginsäure Dinatriumsalz | 50,3 | 9,5 |
| 25 | Verbindung aus Beispiel 1 | 92,4 | 44,5 |
| 26 | Verbindung aus Beispiel 3 | 84,0 | 22,3 |
| 27 | Verbindung aus Beispiel 6 | 81,5 | 15,4 |
| 28 | Verbindung aus Beispiel 8 | 85,4 | 13,6 |

Wie aus Tabelle 4 klar ersichtlich ist, zeigen die erfindungsgemäßen Verbindungen eine bessere biologische Abbaubarkeit und niedrigere Toxizität als die Vergleichsbeispiele aus dem Stand der Technik, insbesondere im Vergleich zum Standard-Quat.

## Patentansprüche

1. Verwendung von Salzen aus Verbindungen der Formel (1) 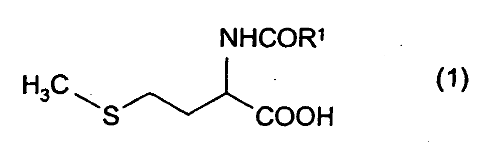 und Aminen der Formel (2) 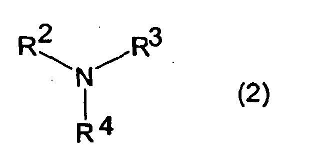 worin
R¹ C₁- bis C₃₀-Alkyl, C₂- bis C₃₀-Alkenyl, C₆- bis C₃₀-Aryl- oder C₇- bis C₃₀- Alkylaryl,
R² C₁- bis C₃₀-Alkyl, C₂- bis C₃₀-Alkenyl, C₆- bis C₃₀-Aryl- oder C₇- bis C₃₀- Alkylaryl, oder einen gegebenenfalls Heteroatome enthaltenden organischen Rest mit 1 bis 30 Kohlenstoffatomen, und,
R³, R⁴ unabhängig voneinander Wasserstoff, C₁- bis C₃₀-Alkyl, C₂- bis C₃₀- Alkenyl, C₆- bis C₃₀-Aryl- oder C₇- bis C₃₀-Alkylaryl, oder einen gegebenenfalls Heteroatome enthaltenden organischen Rest mit 1 bis 30 Kohlenstoffatomen bedeuten, wobei R³ und R⁴ auch unter Einschluss des Stickstoffatoms einen Cyclus mit 5 bis 7 Ringatomen bilden können,
als Korrosionsinhibitoren.

2. Verwendung nach Anspruch wobei R¹ für eine Alkyl- oder Alkenylgruppe von 8 bis 18 Kohlenstoffatomen stehen.

3. Verwendung nach Anspruch 1 und/oder 2, wobei einer, zwei oder alle Reste R², R³ und R⁴ für -CHᵣCH₂-OH stehen.

4. Verwendung nach einem oder mehreren der Ansprüche 1 bis 3, wobei zwei der Reste R², R³, R⁴ für eine C₁- bis C₈-Alkylgruppe und eine Gruppe eine -(CH₂CH₂O)ₙ-H mit n = 2 bis 10 stehen.

5. Verwendung nach Anspruch 1 und/oder 2, worin das Amin der Formel (2) eine Verbindung der Formel (3) ist 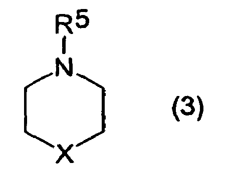 worin
R⁵ Wasserstoff oder einen gegebenenfalls Heteroatome enthaltenden C₁₋₃₀ alkylrest, und
X C, O oder N bedeuten.

6. Verwendung nach einem oder mehreren der Ansprüche 1 bis 5 als Korrosionsinhibitoren an und in Einrichtungen zur Förderung und Transport von Kohlenwasserstoffen in der Erdölförderung und -verarbeitung.

7. Verwendung nach einem oder mehreren der Ansprüche 1 bis 5 als Korrosionsinhibitoren in Metallbearbeitungshilfsmitteln.

## Claims

1. The use of salts of compounds of the formula (1) 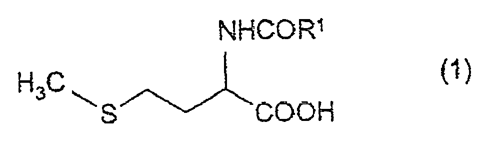 and amines of the formula (2) 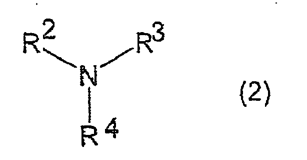 in which
R¹ is C₁- to C₃₀-alkyl, C₂- to C₃₀-alkenyl, C₆- to C₃₀-aryl or C₇- to C₃₀-alkylaryl,
R² is C₁- to C₃₀-alkyl, C₂- to C₃₀-alkenyl, C₆- to C₃₀-aryl or C₇- to C₃₀-alkylaryl, or an organic radical which optionally contains heteroatoms and has from 1 to 30 carbon atoms, and
R³, R⁴ are each independently hydrogen, C₁- to C₃₀-alkyl, C₂- to C₃₀- alkenyl, C₆- to C₃₀-aryl or C₇- to C₃₀-alkylaryl, or an organic radical which optionally contains heteroatoms and has from 1 to 30 carbon atoms, where R³ and R⁴ may also form a cycle with from 5 to 7 ring atoms including the nitrogen atom,
as corrosion inhibitors.

2. The use as claimed in claim 1, wherein R¹ is an alkyl or alkenyl group having from 8 to 18 carbon atoms.

3. The use as claimed in claim 1 and/or 2, wherein one, two or all R², R³ and R⁴ radicals are -CH₂-CH₂-OH.

4. The use as claimed in one or more of claims 1 to 3, wherein two of the R², R³, R⁴ radicals are one C₁- to C₈-alkyl group and one -(CH₂CH₂O)ₙ-H group where n = from 2 to 10.

5. The use as claimed in claim 1 and/or 2, in which the amine of the formula (2) is a compound of the formula (3) 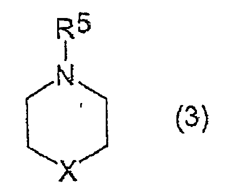 in which
R⁵ is hydrogen or a C₁₋₃₀ alkyl radical which optionally contains heteroatoms, and
X is C, O or N.

6. The use as claimed in one or more of claims 1 to 5 as corrosion inhibitors on and in devices for extraction and transport of hydrocarbons in mineral oil extraction and processing.

7. The use as claimed in one or more of claims 1 to 5 as corrosion inhibitors in metal processing assistants.

## Revendications

1. Utilisation de sels de composés de formule (1) 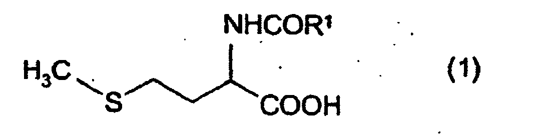 et d'amines de formule (2) 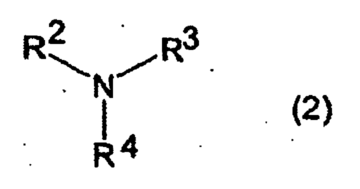 formules dans lesquelles
R¹ représente un groupe alkyle en C₁-C₃₀, alcényle en C₂-C₃₀, aryle en C₆-C₃₀ ou alkylaryle en C₇-C₃₀,
R² représente un groupe alkyle en C₁-C₃₀, alcényle en C₂-C₃₀, aryle en C₆-C₃₀ ou alkylaryle en C₇-C₃₀, ou un radical organique ayant de 1 à 30 atomes de carbone, contenant éventuellement des hétéroatomes, et
R³, R⁴ représentent, indépendamment l'un de l'autre, un atome d'hydrogène, un groupe alkyle en C₁-C₃₀, alcényle en C₂-C₃₀, aryle en C₆-C₃₀ ou alkylaryle en C₇-C₃₀, ou un radical organique ayant de 1 à 30 atomes de carbone, contenant éventuellement des hétéroatomes, R³ et R⁴ pouvant également former, avec inclusion de l'atome d'azote, un cycle ayant de 5 à 7 atomes formant le cycle,
en tant qu'agents anticorrosion.

2. Utilisation selon la revendication 1, dans laquelle R¹ représente un groupe alkyle ou alcényle ayant de 8 à 18 atomes de carbone.

3. Utilisation selon la revendication 1 et/ou la revendication 2, dans laquelle un, deux ou tous les radicaux R², R³ et R⁴ représentent -CH₂-CH₂-OH.

4. Utilisation selon une ou plusieurs des revendications 1 à 3, dans laquelle deux des radicaux R², R³, R⁴ représentent un groupe alkyle en C₁-C₈ et un radical représente un groupe -(CH₂CH₂O)ₙ-H où n = 2 à 10.

5. Utilisation selon la revendication 1 et/ou la revendication 2, dans laquelle l'amine de formule (2) est un composé de formule (3) 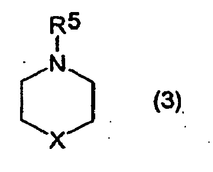 dans laquelle
R⁵ représente un atome d'hydrogène ou un radical alkyle en C₁-C₃₀ contenant éventuellement des hétéroatomes, et
X représente C, O ou N.

6. Utilisation selon une ou plusieurs des revendications 1 à 5, en tant qu'agents anticorrosion sur et dans des dispositifs pour la manutention et le transport d'hydrocarbures dans l'extraction et le traitement du pétrole.

7. Utilisation selon une ou plusieurs des revendications 1 à 5, en tant qu'agents anticorrosion dans des agents auxiliaires pour l'usinage de métaux.
